# EUROPÄISCHE PATENTSCHRIFT

(11) **EP 2 440 904 B1**
(45) Veröffentlichungstag und Bekanntmachung des Hinweises auf die Patenterteilung: **11.04.2018**
(21) Anmeldenummer: 10724304.0
(22) Anmeldetag: 04.06.2010
(51) Int. Cl.: G01N 1/38, G01N 15/06, F16N 39/06, G01N 33/28

(54) **VERFAHREN UND VORRICHTUNG ZUM ERFASSEN VON VERUNREINIGUNGEN IN EINEM FLUID**
METHOD AND DEVICE FOR DETECTING CONTAMINANTS IN A FLUID
PROCÉDÉ ET DISPOSITIF DE DÉTECTION D'IMPURETÉS DANS UN FLUIDE

(30) Priorität: 08.06.2009 DE 102009024561
(43) Veröffentlichungstag der Anmeldung: 18.04.2012
(73) Patentinhaber: Hydac Filter Systems GmbH, 66280 Sulzbach/Saar (DE); SMS group GmbH, 40237 Düsseldorf (DE)
(72) Erfinder: KLEBER, Jörg, 66538 Neunkirchen (DE); GROSS, Sascha, 66346 Püttlingen (DE); MANNEBACH, Horst, 56294 Münstermaifeld (DE); KOHLRAUSCH, Arnt, 57223 Kreuztal (DE); IGELHORST, Wolfgang, 45478 Mülheim a.d. Ruhr (DE)
(74) Vertreter: Bartels und Partner, Patentanwälte
(86) Internationale Anmeldenummer: PCT/EP2010/003389
(87) Internationale Veröffentlichungsnummer: WO 2010/142403

(56) Entgegenhaltungen:
- EP-A1- 0 290 397
- WO-A1-2007/145529
- DE-A1- 10 060 609
- DE-A1-102006 005 956
- FR-A1- 2 448 746
- US-A- 4 583 396
- US-A- 5 586 161
- US-A- 5 739 916

## Beschreibung

Die Erfindung betrifft ein Partikeldetektionsverfahren zum Erfassen oder Detektieren von Verunreinigungen in einem Fluid nach dem Oberbegriff des Anspruches 1, sowie eine Partikeldetektionsvorrichtung zum Durchführen des Verfahrens nach dem Anspruch 4.

Fluide, wie insbesondere Schmieröle oder dergleichen, sind im Betrieb von Anlagen oder Geräten einem ständigen Eintrag von Verunreinigungen in Form von ferromagnetischen Partikeln oder anderen metallischen Partikeln oder sonstigen Schmutzpartikeln ausgesetzt. Dabei ist für den Betrieb einer Anlage oder von Geräten eine gewisse maximale Konzentration an Verunreinigungen je nach Anwendungsfall tolerierbar. Insbesondere bei Walzölen, wie sie in der Stahl- und Schwerindustrie Anwendung finden, werden unter Umständen große Mengen an Verunreinigungen eingetragen, deren Gehalt mit Sensoren nach dem Stand der Technik kaum bestimmbar sind.

Eine derartige Vorrichtung ist beispielsweise in der EP 290 397 B1 beschrieben. Ein Sensor zum Detektieren des Gehalts an ferromagnetischen Partikeln in einem Fluid weist dabei einen Hauptmagnetkreis auf, der durch einen Permanentmagneten gebildet ist. Der Hauptmagnetkreis weist einen Luftspalt auf, der dem Fluid ausgesetzt werden kann, um die Partikel zu veranlassen, sich in der Nähe des Luftspalts zu sammeln. Der Sensor weist ferner ein Hall-Element auf, das in Abhängigkeit von den angesammelten Verunreinigungen die Änderung des magnetischen Flusses detektiert. Es wird an dem Gehalt an Schmutzpartikeln in dem Fluid ein proportionales Detektorsignal erzielt. Ein Hilfsmagnetkreis mit einer Induktionsspule ist vorgesehen, um dem Hauptmagnetkreis des Permanentmagneten entgegenzuwirken, bis hin zu einem völligen Erlöschen des Magnetfelds in dem Luftspalt. Dadurch wird ein Ablösen der in den Luftspalt gesammelten Partikel ermöglicht. Die bekannten Sensoren und Messverfahren zur Bestimmung der Verunreinigung eines Fluides sind jedoch für starken Schmutzeintrag oder Eintrag metallischer Partikel an sich ungeeignet.

In der US 4 583 396 wird ein Kontaminationsniveauindikator aufgezeigt. Aus einem Hydraulikkreis abgezweigtes, verunreinigtes Fluid wird mit Hilfe einer Pumpe einem Filterelement zugeführt. Sodann wird der Druckanstieg stromauf des Filterelements gemessen. Der Druckanstieg ist proportional zur Verschmutzung des Fluids mit Partikeln. Wenn das Filterelement verblockt ist, ist es rückspülbar. Es können auch mehrere Filterelemente vorgesehen sein, mit denen die Messungen wechselweise durchgeführt werden können.

Die WO 2007/145529 A1 betrifft ein Fluidanalysesystem. In einer Bypass-Leitung wird die Molekularstruktur eines Fluids analysiert und mit einer Recheneinheit ausgewertet.

Der Erfindung liegt daher die Aufgabe zugrunde, eine Partikeldetektionsvorrichtung und ein Partikeldetektionsverfahren zum Betrieb der Partikeldetektionsvorrichtung anzugeben, mit welcher sehr hohe Konzentrationen an Verunreinigungen in einem Fluid bestimmbar sind.

Diese Aufgabe wird mit einem Partikeldetektionsverfahren nach dem Anspruch 1 und mit einer Partikeldetektionsvorrichtung nach dem Anspruch 4 gelöst.

Dadurch, dass ein verschmutztes Fluid bevor es an einen Sensor oder eine Vorrichtung zur Messung der Verunreinigung geführt wird, durch eine Zugabe einer definierten Menge an gereinigtem, partikel- und verschmutzungsfreiem Fluid so weit verdünnt wird, dass mit der Vorrichtung die Partikeldichte gemessen werden kann, ist mit bekannten Messverfahren der Grad der Verunreinigung in einem Fluid bestimmbar. Abschließend wird von einer Steuer- und/oder Regelungseinrichtung oder einer Rechnereinheit die Partikeldichte oder der Grad der Verunreinigung des unverdünnten Fluids errechnet. Die Grundstruktur der beiden Fluide ist dabei vorzugsweise gleich.

Bei einem ersten Ausführungsbeispiel wird in einer Mischeinrichtung die sowohl durch einen Behälter als auch durch ein Mischventil darstellbar ist, das verschmutzte und das gereinigte Fluid miteinander gemischt.

In einer Vorrichtung zur Durchführung des Verfahrens ist ein Dosierkreis, also eine hydraulische Anlage vorgesehen, mit welcher das verschmutzte Fluid zu der Vorrichtung zur Messung der Verunreinigungen mittels einer ersten Pumpe oder Dosierpumpe gelenkt wird.

Der Dosierpumpe, die für den Transport von roh-verschmutztem Fluid, wie beispielsweise Walzöl, vorgesehen ist, ist in Fluidrichtung gesehen ein erstes Ventil V₁ nachgeordnet, das vorzugsweise als 3/2-Wegeventil ausgebildet ist. In Abhängigkeit von der Schaltstellung dieses ersten Ventils V₁ kann verschmutztes Fluid entweder in den Dosierkreis zu dem Behälter gelangen oder in Richtung einer Verbindungsstelle des Haupt-Hydrauliksystems weitergeleitet werden.

Des weiteren weist die Vorrichtung einen Hauptkreis auf, der eine zweite Dosierpumpe umfasst, die verschmutztes und/oder gereinigtes Fluid zu einem Sensor fördert. Ein drittes Ventil V₃ und ein viertes Ventil V₄ sind vorgesehen, um zusammen mit den Ventilen des Dosierkreises einen Reinigungsmodus oder einen Mischmodus für gereinigtes und verschmutztes Fluid zu verwirklichen. Ferner kann damit ein Messmodus oder ein Entleermodus für beide Kreise dargestellt werden.

Durch die Steuer- und/oder Regelungseinrichtung lassen sich eine funktionale Kombination von Dosierkreis und Hauptkreis dahingehend bewirken, dass der Dosierkreis in einem Vorbereitungsmodus oder Stand-by-Modus verbleibt und der Hauptkreis in einen Reinigungsmodus zum Abreinigen der Verschmutzungen vor dem Sensor übergeführt wird.

Das angesprochene erfindungsgemäße Verfahren zum Detektieren von Verunreinigungen in einem Fluid läßt sich nun bevorzugt in den folgenden Betriebsarten betreiben. Für das Abreinigen des Öls, das sich im Behälter befindet, ist vorgesehen, dass das erste Ventil V₁ sich in seiner durchgeschalteten Stellung befindet, so dass hochverschmutztes Fluid in Form des Walzöles mittels der ersten Dosierpumpe in Richtung der Verbindungsstelle zum Haupt-Hydrauliksystem hindurchgefördert wird, wobei die erste Dosierpumpe pro Zeiteinheit eine hohe Fluidmenge weiterfördert, so dass Leitungen im Haupt-Hydrauliksystem gespült und Ablagerungen verhindert werden können. Das Walzöl im Umfang seiner aktuellen Verschmutzung wird somit in das Haupt-Hydrauliksystem rückgefördert.

Der insoweit vom Dosierkreis entkoppelte Hauptkreis weist bezogen auf das Ventil V₂ eine durchgehende Schaltstellung auf, so dass die Messvorrichtung umgangen wird. Die Ventile V₃ und V₄ sind derart geschaltet, dass im geschlossenen Kreis Fluid aus dem Behälter durch einen sich an das Ventil V₄ anschließenden Filter hindurchgefördert und derart abgereinigt in den Behälter rückgeführt wird. Auch dergestalt ist die Förderrate der weiteren Dosierpumpe pro Zeiteinheit hoch.

In der Betriebsart "Messen" zwecks Prüfen des Sauberkeitsgehaltes des im Behälter bevorrateten Mischöls ist der Dosierkreis, wie vorstehend dargelegt, vom Hauptkreis wieder entkoppelt und das Ventil V₂ als Umgehungsventil in seiner Sperrstellung gehalten, um dergestalt das Fluid durch die Messvorrichtung hindurchzuleiten. Ferner ist das Ventil V₄ derart geschaltet, dass der Reinigungsfilter umgangen ist. Im dahingehenden Messbetrieb ist die Förderrate der zweiten Dosierpumpe des Hauptkreises klein gewählt.

Im Betriebsmodus "Mischen" und "Dosieren" ist der Dosierkreis, der zum Mischbehälter führt, in Betrieb, d.h. das Ventil V₁ ist derart geschaltet, dass verschmutztes Fluid des Haupt-Hydrauliksystems in den Behälter gelangt. Die Zudosierung erfolgt entweder über eine Taktung des Ventils V₁ oder durch entsprechende Reduzierung der Fluidfördermenge pro Zeiteinheit durch die erste Dosierpumpe. Die weitere Dosierpumpe des Hauptkreises weist wiederum pro Zeiteinheit eine hohe Fluidfördermenge auf und die Messvorrichtung in Form des Contamination-Sensors CS ist wieder über das geschaltete Ventil V₂ umgangen. Die Ventile V₃ und V₄ sind derart geschaltet, dass das Filter wiederum umgangen ist und dergestalt Fluid von dem Ventil V₄ direkt in den Behälter rückgeführt ist.

Die dahingehende Schaltstellung bezogen auf den Hauptkreis entspricht auch dem "Mischen" des Fluids im Hauptkreis, wobei dann der Dosierkreis durch Schalten des Ventils V₁ vom Behälter entkoppelt ist und mit hoher Fluidförderrate durch die erste Dosierpumpe wird wiederum verschmutztes Fluid in das Haupt-Hydrauliksystem weitergefördert. Die letztgenannte Schaltstellung bei entkoppeltem Dosierkreis vom Hauptkreis entspricht auch dem eigentlichen Messvorgang, also der durchzuführenden Messanalyse, bei der jedoch das Ventil V₂ gesperrt ist, so dass mittels der weiteren Dosierpumpe bei geringer Fluidförderrate Fluid durch die Messvorrichtung in Form des Contamination-Sensors geführt wird.

Bei Überschreiten eines Soll-Füllstandes im Behälter kann die Vorrichtung in einem Entleervorgang betrieben werden, bei der unter Umgehung der Messvorrichtung mittels durchgeschaltetem Ventil V₂ Behälterfluid direkt über das geschaltete Ventil V₃ in die Verbindung zum Haupt-Hydrauliksystem gelangt, wobei das Ventil V₄ dann in seiner gesperrten Stellung ist. Die dahingehende Entleerung kann so lange erfolgen, bis im Behälter wiederum die Soll-Füllstandshöhe erreicht ist, die einer minimalen Füllhöhe im Behälter entsprechen kann.

Bei einer weiteren Ausführungsart wird als Sensorelement ein Hall-Element eingesetzt, das an einer für die Menge an Verunreinigungen, insbesondere in Form ferromagnetischer Partikel vorgesehenen Sammelstelle angeordnet ist. Das Sensorelement ermittelt die Menge an Verunreinigungen in dem gemischten Fluid und übermittelt das Ausgangssignal an eine Steuer- und/oder Regelungseinrichtung. Es kann zweckmäßig sein, in der Steuer- und/oder Regelungseinrichtung eine Wertetabelle abzulegen, die einen Zusammenhang zwischen dem Verdünnungsgrad der Menge an Verunreinigung und dem diesbezüglich gegebenenfalls auch stark nicht linearen Ausgangssignal des Sensorelements herstellt. Die Wertetabelle kann dabei auf empirisch ermittelten Werten beruhen. Alternativ hierzu kann in der Steuer- und/oder Regelungseinrichtung auch eine beispielsweise durch Simulation ermittelte Funktion abgelegt sein, die den Zusammenhang zwischen der Menge der angesammelten Verunreinigung und dem Ausgangssignal des Sensors repräsentiert, in Abhängigkeit des Verdünnungsgrads des Fluids.

Die Steuer- und/oder Regelungseinrichtung steuert in Abhängigkeit der gemessenen Werte den Dosierkreis und den Hauptkreis in der Kombination der voranbeschriebenen Betriebsarten. Es kann in diesem Zusammenhang vorteilhaft sein, insbesondere wenn metallische/ferromagnetische Partikel oder Verunreinigungen zu erfassen sind, in der Vorrichtung zur Messung der Verunreinigungen eine Sammelstelle für das Ansammeln der Verunreinigung des Fluids in Form eines Permanentmagneten vorzusehen. Dieser dient zur Erzeugung eines magnetischen Feldes an der Sammelstelle. Eine Spule kann dazu vorgesehen sein, ein Element in der Art eines Ankers derart zu bewegen, dass durch die Bewegung des Elements die magnetische Feldstärke an der Sammelstelle veränderbar ist, insbesondere soweit herabsetzbar ist, dass mindestens eine Teil der angesammelten Verunreinigung von der Sammelstelle ablösbar ist, insbesondere von dem Fluid des Dosierkreises abspülbar ist.

Weitere Vorteile, Merkmale und Einzelheiten der Erfindung ergeben sich aus den Unteransprüchen und der nachfolgenden Beschreibung, in der unter Bezugnahme auf die Zeichnungen ein Ausführungsbeispiel der Erfindung näher beschrieben ist.
Es zeigen in prinzipieller und nicht maßstäblicher Darstellung die
- Fig. 1: einen hydraulischen Schaltplan in der Art einer Funktionsskizze betreffend eine Vorrichtung zum Durchführen eines Verfahrens zum Detektieren von Verunreinigungen;
- Fig. 2: ein Flussdiagramm eines Verfahrens zum Detektieren von Verunreinigungen in einem Fluid, mittels einer Vorrichtung gemäss der Darstellung nach der Fig. 1;
- Fig. 3 bis 5: verschiedene Funktionsskizzen der Vorrichtung nach der Fig.1 in verschiedenen Betriebsarten.

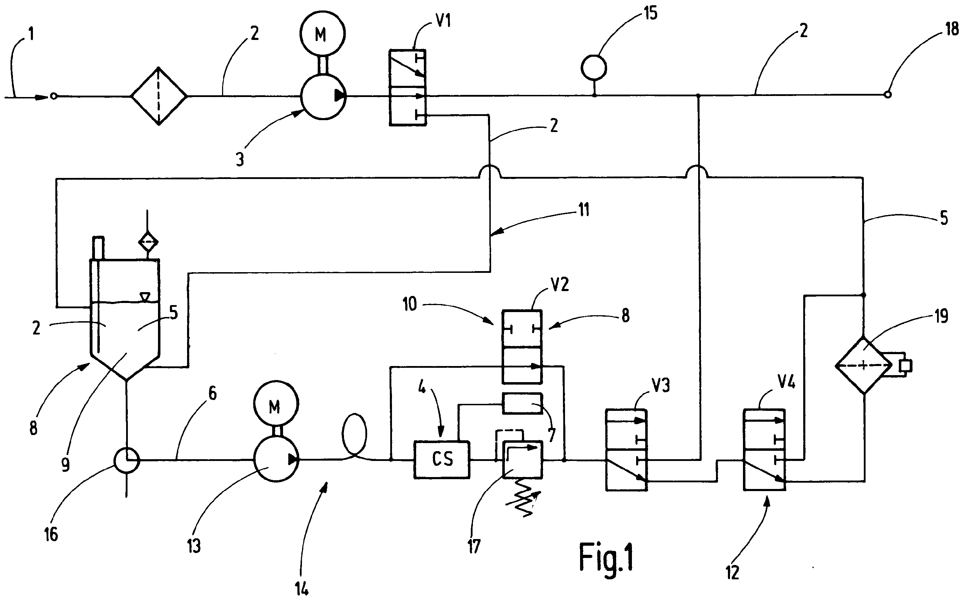

Gemäß der Darstellung nach der Fig.1 ist in einem schematischen Schaltplan eine als Ganzes mit 14 bezeichnete Vorrichtung zur Durchführung eines Messverfahrens zum Detektieren von Verunreinigungen in einem Fluid 1 gezeigt. Das Fluid 1 ist in dem gezeigten Ausführungsbeispiel ein Walzöl einer nicht näher dargestellten Walzstraße oder Walzeinrichtung, das demgemäß hoch verschmutzt ist und mit einem sehr hohen Gehalt an metallischen, insbesondere ferromagnetischen Partikeln nebst anderen Verunreinigungen, wie beispielsweise Schlacke, versehen ist.

Die Vorrichtung 14 besteht im wesentlichen aus einem Dosierkreis 11 und aus einem Hauptkreis 12.

Der Dosierkreis 11 weist eine erste Dosierpumpe 3 mit einem Schrittmotorantrieb auf. Die erste Dosierpumpe 3 fördert verschmutztes Fluid 2 mit Partikeln. Das erste Ventil V₁ ist als 3/2-Wegeventil ausgebildet und in seiner Durchgangsstellung in Fig. 1 gezeigt, in der das verschmutzte Fluid 2 über einen Spänesensor 15 geführt zu einer Verbindung 18 eines Haupthydrauliksystems geführt wird, mithin also im Rücklauf der genannten Walzstraße zugeführt wird. Der genannte Spänesensor 15 ist optional und ist für die eigentliche Funktion der noch näher zu beschreibenden Mess- und Verdünnungseinrichtung nicht zwingend notwendig. Ein drittes Ventil V₃ ist ebenso als 3/2-Wegeventil wie das erste Ventil V₁ ausgebildet. Das dritte Ventil V₃ ist in seiner in Fig. 1 gezeigten Schaltstellung in einer Durchlassstellung zu einem vierten Ventil V₄. Das erste Ventil V₁ ermöglicht in seiner anderen Schaltstellung ferner die Förderung von verschmutztem Fluid 2 in einen Behälter 9, der Teil einer Mischeinrichtung 8 ist, bestehend aus dem Behälter 9 und im vorliegenden Fall aus einem zweiten Ventil V₂, das jedoch für die Grundfunktion der Vorrichtung nicht zwingend benötigt wird.

Der Hauptkreis 12 umfaßt im wesentlichen den Behälter 8, eine Entleerstelle 16 für den Dosierkreis 11, eine zweite Dosierpumpe 13 sowie eine Vorrichtung 4 zur Messung der Verunreinigung des Fluids 1, wobei die Vorrichtung 4 mit einem definierten Gemisch aus gereinigtem Fluid 5 in dem Behälter 8 und verschmutzten Fluid 2, also einer Art gemischtem Fluid 6, beschickt werden kann.

Als Vorrichtung 4 zur Messung der Verunreinigung des Fluids 1 kann ein sogenannter Contamination-Sensor CS eingesetzt werden, wie er beispielsweise in der DE 10 2006 005 956.5 beschrieben ist. Dahingehende Contamination-Sensoren CS arbeiten in der Art von Partikelsensoren auf Lichtbasis, d.h. die Partikel werden nach Durchlaufen einer Lichtschranke od. dgl. nach Größe und Anzahl bestimmt, so dass dem noch näher zu erläuterndem Verdünnungsaspekt des Messfluids erhöhte Bedeutung zukommt. Erst durch das angesprochene erfindungsgemäße Verdünnen oder Dekonzentrieren des Fluidmediums im vorgebbaren einstellbaren Rahmen sind die zu detektierenden Partikel in einer statistischen Verteilung im Fluid derart vereinzelt vorhanden, dass die lichtbasierten Sensoren überhaupt ansprechen können. Ohne diese Vereinzelung durch Verdünnung könnte nur ein von den Partikeln eingetrübtes Fluid festgestellt werden ohne Aussagegehalt darüber, wie sich der Verschmutzungsgrad im einzelnen tatsächlich darstellt wegen der fehlenden Zählgröße betreffend die generelle Partikelverschmutzung. Die vorzunehmende Verdünnung orientiert sich dabei vorrangig an der Messqualität des jeweils einzusetzenden Lichtsensors.

Ein der Vorrichtung 4 zur Messung der Verunreinigungen nachgelagertes Druckregelventil 17 sorgt für einen blasenfreien vorgespannten Betrieb der Vorrichtung 4. Des weiteren ist zwischen der zweiten Dosierpumpe 13 und dem Contamination-Sensor CS in der Art einer Schlaufe eine Einlaufstrecke für das Fluid dargestellt, dessen Länge beliebig vorgebbar ist. Das zweite Ventil V₂, das als 2/2-Wegeventil ausgebildet ist, dient als Umgehungs- oder Mischventil 10 und ermöglicht insoweit die Umgehung der Vorrichtung 4 und des Druckregelventils 17. In Verbindung mit der in der Fig.1 dargestellten Schaltstellung des dritten Ventils V₃ und des vierten Ventils V₄, gleichfalls als 3/2-Wegeventil ausgebildet, ist ein Rückfluß von gemischtem Fluid 6 in den Behälter 8 möglich, wobei in Abhängigkeit der Schaltstellung des vierten Ventils V₄ eine Durchströmung eines zusätzlichen Filters 19 zum Abreinigen des Fluids erfolgen kann oder nicht. In der gezeigten Schaltdarstellung nach der Fig.1 wird aber jedenfalls der Filter 19 durchströmt. Das zweite Ventil V₂ ist nicht unbedingt notwendig und bei dessen Weglassen werden in jedem Fall der Contamination-Sensor CS und das Druckregelventil 17 durchströmt.

Das erste, dritte und vierte Ventil V₁, V₃ und V₄ sind als 3/2-Wegeventile ausgebildet, so dass die insofern eingesetzten Ventilbaugruppen als Gleichbauteile ausgebildet sind, was den vorrichtungsgemäßen Aufwand reduzieren hilft, so dass die Gesamtvorrichtung kostengünstig herstellbar ist. Alle Ventile lassen sich vorzugsweise elektromagnetisch ansteuern, was der einfacheren Darstellung wegen aber nicht näher gezeigt ist.

Eine Recheneinheit 7, die Teil einer nicht näher dargestellten Steuer- und/oder Regelungseinrichtung oder Teil der Vorrichtung 4 zur Messung der Verunreinigung in dem Fluid sein kann, rechnet den von der Vorrichtung 4 ermittelten Gehalt an Schmutzpartikeln zurück auf den tatsächlichen Gehalt an Schmutzpartikeln in dem nicht gemischten Fluid 2 mit der Partikelverschmutzung aus dem Haupt-Hydrauliksystem, das im vorliegenden Ausführungsbeispiel das Walzöl der Walzstraße transportiert. Anstelle des genannten Walzöls kann natürlich auch jede andere Form mehr oder minder stark verschmutzten Fluids mit Partikeleintrag über das beschriebene Verfahren nebst Vorrichtung behandelt werden.

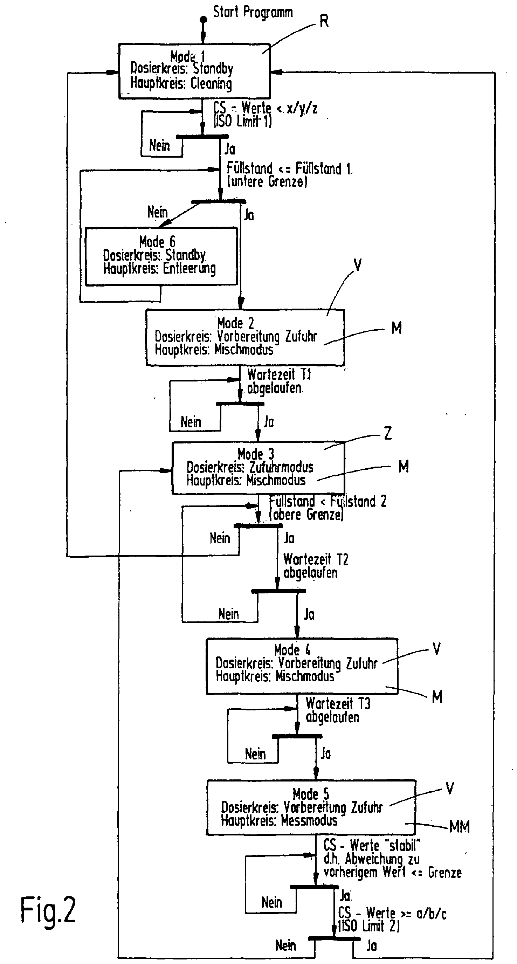

In der Fig.2 ist in der Art eines Flussdiagramms eine von vielen Betriebsweisen oder Verfahren zum Detektieren von Verunreinigungen mit der in Fig.1 gezeigten Vorrichtung 14 dargestellt. Im Rahmen einer ersten Betriebsweise (Mode 1) ist der Dosierkreis 11 in einem Standby-Modus und der Hauptkreis 12 befindet sich in einem Reinigungsmodus R (cleaning).

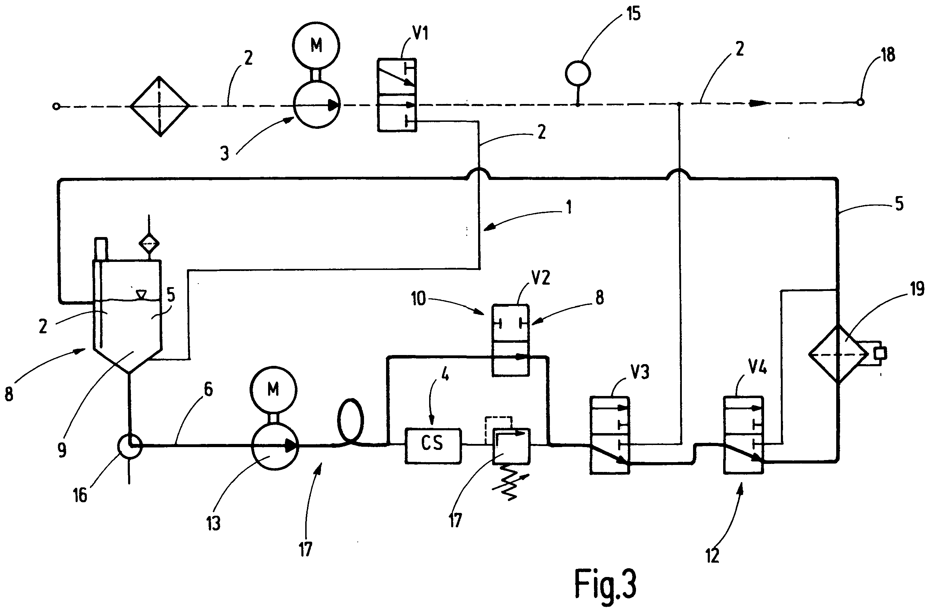

Eine weitere Betriebssituation "Abreinigung des Öls" ist in der Fig.3 verdeutlicht und im strichliniert dargestellten Leitungsteil wird bevorzugt eine große Fluidmenge mittels des Schrittmotorantriebs und der Dosierpumpe 3 durch den Arbeitskreis gebracht, um dergestalt beispielsweise die angeschlossenen Leitungen zu spülen, was Ablagerungen verhindern hilft, wobei Fluid mit aktueller Verschmutzung über die Verbindung 18 in das Haupt-Hydrauliksystem von der Eingangsseite her weiter zugeführt wird. Hierbei befindet sich das Ventil V₁ in seiner gezeigten durchgeschalteten Stellung.

Der Hauptkreis 12 (in verstärkt durchgezogener Linie dargestellt) wird mit gereinigtem Fluid, das über den Filter 19 demgemäß geführt ist, versorgt. Dabei ist das zweite Ventil V₂, das dritte und das vierte Ventil V₃, V₄ derart in einer gezeigten Schaltstellung, dass der von der zweiten Dosierpumpe 13 erzeugte Fluidstrom an dem Sensor CS oder der Vorrichtung 4 vorbei durch das dritte Ventil V₃ und vierte Ventil V₄ geführt ist und insoweit in den Behälter 9 in einer geschlossenen Kreislaufführung rückgeführt werden kann. Dieser Kreislauf wiederholt sich, bis ein definierter Reinheitsgrad für das Fluid 5 erreicht ist. Beim dahingehenden Abreinigungsvorgang des Öls über den Filter 19 ist der Schrittmotorantrieb mit der zweiten Dosierpumpe 13 im Bereich großer Fluidmengen pro Zeiteinheit im Hauptkreis 12 betrieben.

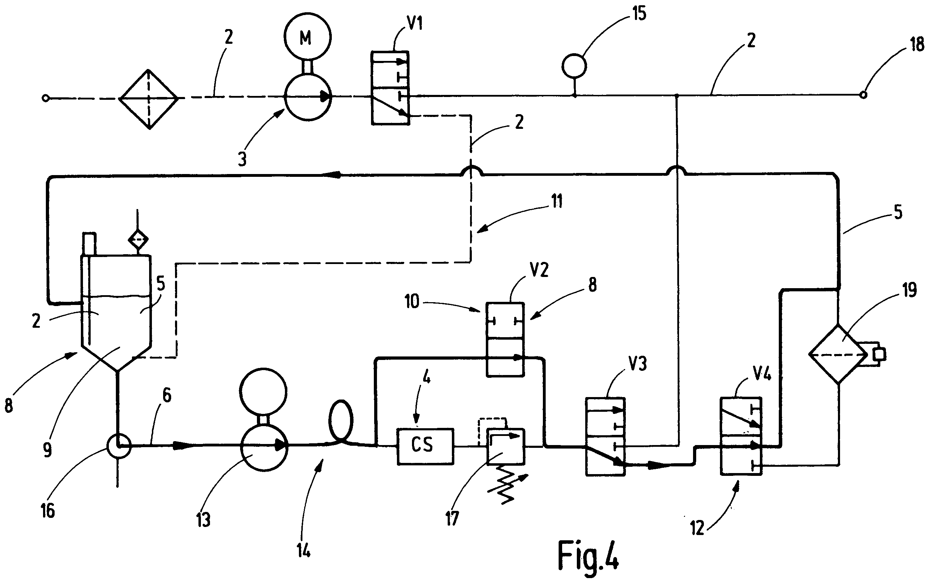

Im reinen Mischbetrieb zur Mischung von gereinigtem Fluid 5 mit verschmutztem Fluid 2 ist das Ventil V₄ derart geschaltet, dass dann der Filter 19 umgangen ist. Ferner kann in einen Modus 2 (Mode 2) oder Mischmodus M gewechselt werden, indem gereinigtes Fluid 5 mit verschmutzten Fluid 2 definiert gemischt wird, was weitgehend der Darstellung nach der Fig.4 entspricht, die einen "Misch- und Dosiervorgang" zeigt. Der Dosierkreis 11 (strichliniert dargestellt) wechselt in einen vorbereiteten Zuführmodus V, in dem verschmutztes Fluid 2 der Mischeinrichtung 8, hier in Form des Behälters 9, zugeführt wird. Für einen besonders günstigen Verfahrensablauf hat es sich herausgestellt, die Verbindungsleitung zwischen dem Behälter 9 und dem Ventil V₁ besonders kurz zu dimensionieren, beispielsweise kleiner als 10 cm Leitungslänge zu wählen. Für den dahingehenden Zudosier- oder Zumischvorgang fördert die Dosierpumpe 3 nur eine geringe Menge an verschmutztem Fluid 2 pro Zeiteinheit, wobei anstelle einer niedrigen Zudosierung über die Pumpe 3 auch das Ventil V₁ in einen entsprechend getakteten Betrieb gehen kann, bei dem in Schritten immer eine bestimmte Portionsmenge aus der Hauptleitung mit dem Walzöl in den Dosierkreis 11 übernommen wird. Im dahingehenden Fall übernimmt die Recheneinheit 7 den getakteten Betrieb für das Ventil V₁.

Das dritte Ventil V₃ und das vierte Ventil V₄ sind dabei in einer gezeigten Schaltstellung nach der Fig.4, bei der der Filter 19 in den dahingehenden Mode übergangen ist, so dass Fluid von der zweiten Dosierpumpe 13 wiederum bei einer hohen Durchflußmenge pro Zeiteinheit zu der Mischeinrichtung 8 geführt ist.

Nach einer vorgebbaren Warte- oder Beruhigungszeit wird in einen Modus 3 (Mode 3) gewechselt, in dem der Dosierkreis 11 in einem Zuführmodus Z für verschmutztes Fluid 2 zu der Mischeinrichtung 8 ist und der Hauptkreis 12 (wiederum in verstärkt durchgezogener Linie dargestellt) in einem Mischmodus M verbleibt. Die Schaltstellung des ersten Ventils V₁ ist dabei derart, dass verschmutztes Fluid 2 in die Mischeinrichtung 8 gefördert wird. Die Schaltstellung des zweiten Ventils V₂, des dritten und vierten Ventils V₃, V₄ ist, wie wiederum in Fig. 4 dargestellt, derart, dass gemischtes Fluid 6 an der Vorrichtung 4 zur Messung der Verunreinigungen vorbei zu der Mischeinrichtung 8 im geschlossenen Kreis bewegt wird.

Je nach gewünschter Reinheitsklasse sind die bevorzugten Mischungsverhältnisse zwischen verschmutztem Fluid 2 und gereinigtem Fluid 5 bevorzugt zwischen 1 : 10 bis etwa 1 : 150 eingestellt, d.h. beispielsweise dass ein Milliliter verschmutztes Fluid 2 auf 10 Milliliter gereinigtes Fluid 5 kommt, wobei für einen dahingehenden Zumischvorgang an verunreinigtem Fluid über den Dosierkreis 11 im Behälter 9 vorab eine vorgebbare Menge an gereinigtem Fluid 5 sich bereits befinden muß. Zur Erläuterung sei noch erwähnt, dass die Füllstandshöhe in dem Behälter 9 in den Figuren die minimale Füllhöhe des Behälters 9 betrifft.

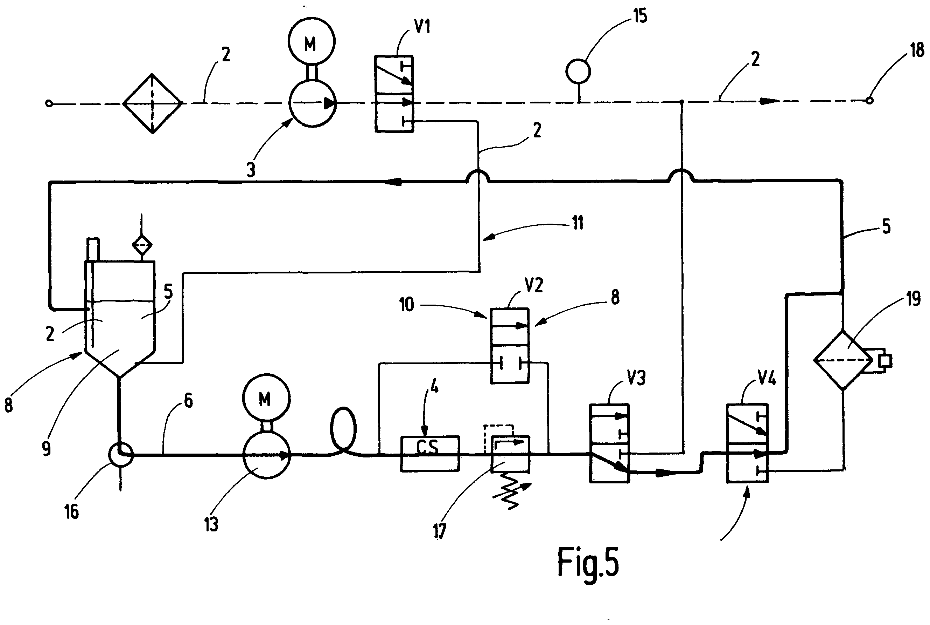

Nach erneutem Ablauf einer vorgebbaren Wartezeit wechselt das Verfahren im Hauptkreis 12 (wiederum in verstärkt durchgezogener Linie dargestellt) in einen sog. Messmodus MM (Mode 5). Im dahingehenden Messbetrieb, was der Schaltdarstellung nach der Fig. 5 entspricht, ist der Dosierkreis 11 in einer Betriebsweise, indem vergleichbar der Darstellung nach der Fig.2 verschmutztes Fluid direkt von der Eingangsseite zu dem Haupt-Hydrauliksystem über die Verbindung 18 geführt ist. Der dahingehende Hauptkreisverlauf ist wiederum strichliniert dargestellt. Dabei ist das zweite Ventil V₂ in seiner gezeigten Sperrstellung und das vierte Ventil V₄ und das dritte Ventil V₃ in ihrer gezeigten Schaltstellung, in der gemischtes Fluid 6 durch die Vorrichtung 4 zur Messung der Verunreinigung geführt ist, wobei jedoch der Filter 19 ausgelassen bleibt. Die Dosierpumpe 3 fördert wiederum pro Zeiteinheit eine große Fluidmenge und die Dosierpumpe 13 eine kleine Menge. Die benötigten Messwerte werden im einzelnen ermittelt, sobald deren Varianz eine bestimmte Grenze nicht überschreitet.

Wie Fig.2 des weiteren zeigt, ist zwischen dem Modus 5 und dem Modus 3 noch eine Betriebsweise (Mode 4) geschaltet, bei der der Dosierkreis 11 in Vorbereitung Zufuhr V und Hautpkreis 12 in den Mischmodus M gebracht ist. Im Modus 6 (Mode 6) befindet sich der Dosierkreis 11 im Standby-Modus. Bei einer weiter vorgesehenen Entleerung wird ein Soll-Füllstand im Behälter 9 angestrebt und in einer durchgeschalteten Schaltstellung des Ventils V₃ wird die Fluidmenge des Hauptkreises 12 über die Verbindung 18 als Rücklauf in das Haupt-Hydrauliksystem zurückgeführt. Anschließend kann wieder in den Modus 1 als Betriebsart gewechselt.

Bei einer vereinfachten, nicht näher dargestellten Ausführungsform des erfindungsgemäßen Verfahrens nebst Vorrichtung kann der Behälter 9 nebst der Entleerstelle 16 auch entfallen und das Ventil V₁ ist ausgangsseitig über den Dosierkreis 11 direkt auf die Eingangsseite der zweiten Dosierpumpe 13 geschaltet. Im dahingehenden Fall ist wiederum die Verbindungsleitung zwischen dem Ventil V₁ und der Dosierpumpe 13 kurz zu wählen und gleichfalls wird eine kurze Verbindung zwischen dem Ausgang der zweiten Dosierpumpe 13 und dem Eingang des Contamination-Sensors CS angestrebt. Bei der dahingehend vereinfachten Ausführungsform kann ferner das Umgehungs- oder Mischventil V₂ entfallen ebenso wie das Druckregelventil 17. Wie bereits dargelegt, kann auch der Spänesensor 15 entfallen. Die benötigte Zumischung erfolgt dann, wie bereits dargelegt, über das Zuschalten der Dosierpumpe 3 und bevorzugt durch einen getakteten Betrieb des Ventils V₁, das dann verschmutztes Fluid 2 portionsweise in den Dosierkreis 11 überführen kann.

Bei einer weiter vereinfachten und nicht dargestellten Ausführungsform wird das verunreinigte Fluid mit der Partikelverschmutzung, wie beispielsweise Walzöl, über die Dosierpumpe 3 mengenmäßig gesteuert direkt an den Eingang der zweiten Dosierpumpe 13 geführt, die auf ihrer Ausgangsseite dann die Vorrichtung 4 zur Messung der Vernreinigungen aufweist, wobei in einem Abzweig vor der zweiten Dosierpumpe 13 diese Frischöl aus einer Versorgungsquelle, wie beispielsweise einem Faß, fördern kann. Die dahingehende Einfachstlösung kommt ohne jede Ventilsteuerung aus und mithin ohne anzusteuernde Ventile. Das derart über den Contamination-Sensor CS gemessene Fluid wird dann wiederum in das Haupt-Hydrauliksystem, beispielsweise in Form der Walzstraße, rückgeführt.

Anstelle des Behälters 9 oder dem genannten Abzweig zwischen den beiden Dosierpumpen 3 und 13 kann auch ein nicht näher bestimmtes Mischventil treten, das dann wiederum über die Recheneinheit 7 angesteuert die Zumischung übernimmt. Des weiteren kann bei einer nicht näher dargestellten Ausführungsform der Mess- und Zumischvorrichtung der Dosierkreis 11 mit einer Zuführleitung direkt vom Ventil V₁ kommend vor den Contamination-Sensor CS geschaltet sein, was eine Integration der Vorrichtung in allfällige Ventilblöcke erleichtern hilft. Die Zuführleitung endet also hinter der weiteren Dosierpumpe 13 in die Fluidstrecke zum Sensor CS.

## Patentansprüche

1. Partikeldetektionsverfahren zum Detektieren von Verunreinigungen in einem Fluid (1), wobei das mit Partikeln verschmutzte Fluid (2) mit einer ersten Dosierpumpe (3) zu einer Vorrichtung (4) zur Messung der Verunreinigung oder Partikeldichte in dem verschmutzten Fluid gefördert wird, **dadurch gekennzeichnet, dass** das mit den Partikeln verschmutzte Fluid (2) vor dem Eintritt in die Vorrichtung (4) zur Messung der Verunreinigung mit gereinigtem Fluid (5) in einem definierten Mischungsverhältnis gemischt, die Partikeldichte oder die Verunreinigung des gemischten Fluids (6) gemessen, und von einer Recheneinheit (7) die Partikeldichte oder die Verunreinigung des verschmutzten Fluids (2) bestimmt wird.

2. Partikeldetektionsverfahren zum Detektieren von Verunreinigungen in einem Fluid (1) nach Anspruch 1, **dadurch gekennzeichnet, dass** das verschmutzte Fluid (2) und das gereinigte Fluid (5) in einer Mischeinrichtung (8) miteinander gemischt werden.

3. Partikeldetektionsverfahren zum Detektieren von Verunreinigungen in einem Fluid (1) nach Anspruch 2, **dadurch gekennzeichnet, dass** die Mischeinrichtung (8) ein Behälter (9) und/oder ein Mischventil ist.

4. Partikeldetektionsvorrichtung zum Durchführen des Partikeldetektionsverfahrens nach einem der Ansprüche 1 bis 3, wobei das mit Partikeln verschmutzte Fluid (2) mit einer ersten Dosierpumpe (3) zu einer Vorrichtung (4) zur Messung der Verunreinigung oder Partikeldichte in dem verschmutzten Fluid förderbar ist, **dadurch gekennzeichnet, dass** das mit den Partikeln verschmutzte Fluid (2) vor dem Eintritt in die Vorrichtung (4) zur Messung der Verunreinigung oder Partikeldichte mit gereinigtem Fluid (5) in einem definierten Mischungsverhältnis mischbar ist, dass die Partikeldichte oder die Verunreinigung des gemischten Fluids (6) mit der Vorrichtung (4) zur Messung der Verunreinigung oder Partikeldichte messbar ist und von einer Recheneinheit (7) die Partikeldichte oder die Verunreinigung des verschmutzten Fluids (2) bestimmbar ist, und dass ein Dosierkreis (11) vorgesehen ist, mit dem verschmutztes Fluid (2) zu der Vorrichtung (4) zur Messung der Verunreinigungen durch die erste Dosierpumpe (3) förderbar ist, wobei ein der ersten Dosierpumpe (3) nachgelagertes, erstes Ventil (V₁) vorgesehen ist, und wobei mit dem Dosierkreis (11) in Abhängigkeit von der Schaltstellung des Ventils (V₁) verschmutztes Fluid (2) entweder in einen Behälter (9) oder in Richtung einer Verbindung (18) zu einem Haupt-Hydrauliksystem förderbar ist.

5. Partikeldetektionsvorrichtung nach Anspruch 4, **dadurch gekennzeichnet, dass** ein Hauptkreis (12) eine zweite Dosierpumpe (13) umfasst, die verschmutztes und gereinigtes Fluid (2, 5) zu der Vorrichtung (4) zur Messung der Verunreinigung fördert, wobei ein drittes Ventil (V₃) und ein viertes Ventil (V₄) vorgesehen sind und in Abhängigkeit von der Schaltstellung der Ventile (V₃, V₄) des Hauptkreises (12) entweder ein Reinigungsmodus (R) oder ein Mischmodus (M) für gereinigtes und verschmutztes Fluid (5, 2) oder ein Messmodus (MM) oder ein Entleermodus für den Dosierkreis (11) und/oder den Hauptkreis (12) betrieben werden kann.

6. Partikeldetektionsvorrichtung nach Anspruch 5, **dadurch gekennzeichnet, dass** eine Kombination des Dosierkreises (11) und des Hauptkreises (12) derart vorgesehen ist, dass der Dosierkreis (11) in einem Standby-Modus und der Hauptkreis (12) in einem Reinigungsmodus (R) betreibbar ist.

7. Partikeldetektionsvorrichtung nach Anspruch 6, **dadurch gekennzeichnet, dass** die erste Dosierpumpe (3) in Betrieb ist, das erste Ventil (V₁) in einer Schaltstellung sich befindet, in der eine fluidführende Verbindung mit dem Haupt-Hydrauliksystem hergestellt ist, und das zweite, dritte und vierte Ventil (V₂, V₃, V₄) derart eine Schaltstellung aufweisen, dass ein Fluidstrom mit gereinigtem Fluid (5) von dem Behälter (9) über die zweite Dosierpumpe (13) zu dem zweiten Ventil (V₂) und über das dritte und vierte Ventil (V₃, V₄) geführt zu einem Filter (19) gelangt und wieder zu dem Behälter (9) rückgeführt ist.

8. Partikeldetektionsvorrichtung nach Anspruch 5, **dadurch gekennzeichnet, dass** eine Kombination des Dosierkreises (11) und des Hauptkreises (12) derart vorgesehen ist, dass der Dosierkreis (11) in einem Vorbereitungsmodus (V) zum Zuführen von verschmutztem Fluid (2) in die Mischeinrichtung (8) und der Hauptkreis (12) in einem Mischmodus (M) für verschmutztes und gereinigtes Fluid (2, 5) ist.

9. Partikeldetektionsvorrichtung nach Anspruch 8, **dadurch gekennzeichnet, dass** die erste Dosierpumpe (3) in Betrieb ist und das erste Ventil (V₁) in einer Schaltstellung ist, in der verschmutztes Fluid (2) zu dem Behälter (9) geführt ist, wobei das zweite Ventil (V₂) sowie das dritte Ventil (V₃) und das vierte Ventil (V₄) in einer Schaltstellung sind, dass ein Fluidstrom von der zweiten Dosierpumpe (13) zu der Mischeinrichtung (8) unter Umgehung der Vorrichtung (4) zur Messung der Verschmutzung des Fluids (1) sowie unter Umgehung des Filters (19) geführt ist.

10. Partikeldetektionsvorrichtung nach Anspruch 5, **dadurch gekennzeichnet, dass** eine Kombination des Dosierkreises (11) und des Hauptkreises (12) derart vorgesehen ist, dass der Dosierkreis (11) in einem Zuführmodus (Z) für verschmutztes Fluid (2) und der Hauptkreis (12) in einem Mischmodus (M) für verschmutztes Fluid (2) und gereinigtes Fluid (5) ist.

11. Partikeldetektionsvorrichtung nach Anspruch 10, **dadurch gekennzeichnet, dass** die erste Dosierpumpe (3) in Betrieb ist und das erste Ventil (V₁) das verschmutzte Fluid (2) in die Mischeinrichtung (8) fördert und die Schaltstellung des zweiten Ventils (V₂), des dritten Ventils (V₃) und des vierten Ventils (V₄) derart ist, dass das gemischte Fluid (6) an der Vorrichtung (4) zur Messung der Verunreinigung vorbei von der Mischeinrichtung (8) wieder zu dieser Mischeinrichtung (8) geführt ist.

12. Partikeldetektionsvorrichtung nach Anspruch 5, **dadurch gekennzeichnet, dass** eine Kombination des Dosierkreises (11) und des Hauptkreises (12) derart vorgesehen ist, dass der Dosierkreis (11) in einem Vorbereitungsmodus (V) zum Zuführen verschmutzten Fluids (2) in die Mischeinrichtung (8) und der Hauptkreis (12) in einem Messmodus (MM) ist.

13. Partikeldetektionsvorrichtung nach Anspruch 12, **dadurch gekennzeichnet, dass** die erste Dosierpumpe (3) in Betrieb ist und durch das erste Ventil (V₁) das verschmutzte Fluid (2) zu dem Haupt-Hydrauliksystem geführt ist, wobei das zweite Ventil (V₂) in einer Sperrstellung ist, so dass gemischtes Fluid (6) durch die Vorrichtung (4) zur Messung der Verunreinigung geführt ist und das dritte Ventil (V₃) sowie das vierte Ventil (V₄) sind in einer Schaltstellung, dass gemessenes Fluid in den Behälter (9) rückgeführt ist.

14. Partikeldetektionsvorrichtung nach Anspruch 5, **dadurch gekennzeichnet, dass** eine Kombination des Dosierkreises (11) und des Hauptkreises (12) derart vorgesehen ist, dass das verschmutzte Fluid (2) im Dosierkreis (11) nicht bewegt ist und das gereinigte und/oder gemischte Fluid (5, 6) aus der Mischeinrichtung (8) in den Hauptkreis (12) entleerbar ist.

15. Partikeldetektionsvorrichtung nach Anspruch 14, **dadurch gekennzeichnet, dass** das zweite Ventil (V₂) eine solche Schaltstellung einnimmt, dass verschmutztes Fluid (2) zu dem dritten Ventil (V₃) geführt ist und von dem dritten Ventil (V₃) das Fluid (2) zu dem Haupt-Hydrauliksystem über die Verbindung (18) geführt ist, wobei das vierte Ventil (V₄) eine sperrende Schaltstellung einnimmt, das eine Entleerung des Dosierkreises (11) und des Hauptkreises (12) ermöglicht, wobei das Ventil (V₁) verschmutztes Fluid gleichfalls in die Verbindung (18) des Haupt-Hydrauliksystems durchläßt.

16. Partikeldetektionsvorrichtung nach einem der Ansprüche 4 bis 15, **dadurch gekennzeichnet, dass** die Vorrichtung (14) ein Sensorelement (CS), wie etwa einen optischen oder Hall-Sensor aufweist, das die Menge der Verschmutzungspartikel ermittelt und an eine Steuer- und/oder Regelungseinrichtung übermittelt ist, die den Dosierkreis (11) und den Hauptkreis (12) mittels der Ventile (V₁, V₂, V₃, V₄) sowie der Dosierpumpen (3, 13) steuert.

17. Partikeldetektionsvorrichtung nach einem der Ansprüche 7 bis 16, **dadurch gekennzeichnet, dass** die Vorrichtung (14) eine Sammelstelle für das Ansammeln von Verunreinigungen aufweist und einen Permanentmagneten zum Erzeugen eines magnetischen Feldes an der Sammelstelle und eine Spule hat, wobei durch das Bestromen der Spule eine Element der Vorrichtung (14) bewegbar ist und durch die Bewegung des Elements die magnetische Feldstärke an der Sammelstelle veränderbar ist.

## Claims

1. A particle detection method for detecting contaminants in a fluid (1), the fluid (2) contaminated with particles being conveyed by a first metering pump (3) to a device (4) for measuring the contamination or particle density in the contaminated fluid, **characterised in that** before entering the device (4) for measuring the contamination, the fluid (2) contaminated with the particles is mixed with cleaned fluid (5) in a defined mixing ratio, the particle density or the contamination of the mixed fluid (6) is measured, and the particle density or the contamination of the contaminated fluid (2) is determined by a computing unit (7).

2. The particle detection method for detecting contaminants in a fluid (1) according to Claim 1, **characterised in that** the contaminated fluid (2) and the cleaned fluid (5) are mixed with one another in a mixing device (8).

3. The particle detection method for detecting contaminants in a fluid (1) according to Claim 2, **characterised in that** the mixing device (8) is a tank (9) and/or a mixing valve.

4. The particle detection device for implementing the particle detection method according to any of Claims 1 to 3, the fluid (2) contaminated with particles being able to be conveyed by a first metering pump (3) to a device (4) for measuring the contamination or particle density in the contaminated fluid, **characterised in that** before entering the device (4) for measuring the contamination or the particle density, the fluid (2) contaminated with particles is able to be mixed with cleaned fluid (5) in a defined mixing ratio, that the particle density or the contamination of the mixed fluid (6) can be measured with the device (4) for measuring the contamination or the particle density, and the particle density or the contamination of the contaminated fluid (2) can be determined by a computing unit (7), and that a metering circuit (11) is provided with which contaminated fluid (2) can be routed to the device (4) for measuring the contaminants by means of the first metering pump (3), a first valve (V₁) being provided downstream of the first metering pump (3), and depending on the switching position of the valve (V₁), the metering circuit (11) being able to convey contaminated fluid (2) either into a tank (9) or in the direction of a connection (18) to a main hydraulic system.

5. The particle detection device according to Claim 4, **characterised in that** a primary circuit (12) comprises a second metering pump (13) which delivers contaminated and cleaned fluid (2, 5) to the device for measuring the contamination, a third valve (V₃) and a fourth valve (V₄) being provided and, depending on the switching position of the valves (V₃, V₄) of the primary circuit (12), either a cleaning mode (R) or a mixing mode (M) for cleaned and contaminated fluid (5, 2) or a measuring mode (MM) or an evacuation mode for the metering circuit (11) and/or the primary circuit (12) being able to be operated.

6. The particle detection device according to Claim 5, **characterised in that** a combination of the metering circuit (11) and the primary circuit (12) is provided such that the metering circuit (11) remains in a standby mode and the primary circuit (12) can be operated in a cleaning mode (R).

7. The particle detection device according to Claim 6, **characterised in that** the first metering pump (3) is in operation, the first valve (V₁) is in a switching position in which a fluid-conveying connection to the main hydraulic system is established, and the second, third and fourth valves (V₂, V₃, V₄) have a switching position such that a fluid flow with cleaned fluid (5) passes from the tank (9) via the second metering pump (13) to the second valve (V₂) and is routed via the third and the fourth valve (V₃, V₄) to a filter (19) and is returned again to the tank (9).

8. The particle detection device according to Claim 5, **characterised in that** a combination of the metering circuit (11) and of the primary circuit (12) is provided such that the metering circuit (11) is in a preparation mode (V) for supplying contaminated fluid (2) to the mixing device (8) and the primary circuit (12) is in a mixing mode (M) for contaminated and cleaned fluid (2, 5).

9. The particle detection device according to Claim 8, **characterised in that** the first metering pump (3) is in operation and the first valve (V₁) is in a switching position in which contaminated fluid (2) is routed to the tank (9), the second valve (V₂) and the third valve (V₃) and the fourth valve (V₄) being in one switching position, that a fluid flow is routed from the second metering pump (13) to the mixing device (8), bypassing the device (4) for measuring the contamination of the fluid (1) and bypassing the filter (19).

10. The particle detection device according to Claim 5, **characterised in that** a combination of the metering circuit (11) and of the primary circuit (12) is provided such that the metering circuit (11) is in a supply mode (Z) for contaminated fluid (2) and the primary circuit (12) is in a mixing mode (M) for contaminated fluid (2) and cleaned fluid (5).

11. The particle detection device according to Claim 10, **characterised in that** the first metering pump (3) is in operation and the first valve (V₁) conveys the contaminated fluid (2) into the mixing device (8) and the switching position of the second valve (V₂), the third valve (V₃) and the fourth valve (V₄) is such that the mixed fluid (6) on the device (4) for measuring the contamination is routed past the mixing device (8) again to this mixing device (8).

12. The particle detection device according to Claim 5, **characterised in that** a combination of the metering circuit (11) and of the primary circuit (12) is provided such that the metering circuit (11) is in a preparation mode (V) for supplying contaminated fluid (2) to the mixing device (8) and the primary circuit (12) is in a measuring mode (MM).

13. The particle detection device according to Claim 12, **characterised in that** the first metering pump (3) is in operation and the first valve (V₁) routes the contaminated fluid (2) to the main hydraulic system, the second valve (V₂) being in a blocking position so that mixed fluid (6) is routed through the device (4) for measuring the contamination and the third valve (V₃) and the fourth valve (V₄) are in one switching position, and that measured fluid is returned to the tank (9).

14. The particle detection device according to Claim 5, **characterised in that** a combination of the metering circuit (11) and of the primary circuit (12) is provided such that the contaminated fluid (2) in the metering circuit (11) is not moved and the cleaned and/or mixed fluid (5, 6) can be evacuated from the mixing device (8) into the primary circuit (12).

15. The particle detection device according to Claim 14, **characterised in that** the second valve (V₂) assumes a switching position such that contaminated fluid (2) is routed to the third valve (V₃), and from the third valve (V₃) the fluid (2) is routed to the main hydraulic system via the connection (18), the fourth valve (V₄) assuming a blocking switching position, which valve enables evacuation of the metering circuit (11) and of the main circuit (12), the valve (V₁) likewise allowing contaminated fluid to pass into the connection (18) of the main hydraulic system.

16. The particle detection device according to any of Claims 4 to 15, **characterised in that** the device (14) has a sensor element (CS) such as, for example, an optical or Hall sensor which determines the quantity of contamination particles and transmits this to a control and/or regulation device which controls the metering circuit (11) and the primary circuit (12) by means of the valves (V₁, V₂, V₃, V₄) and the metering pumps (3, 13).

17. The particle detection device according to any of Claims 7 to 16, **characterised in that** the device (14) has a collecting site for collecting contaminants and at the collecting site has a permanent magnet for generating a magnetic field and a coil, by energising the coil, an element of the device (14) being able to be moved, and by moving the element, the magnetic field strength at the collecting site being able to the changed.

## Revendications

1. Procédé de détection de particules, pour détecter des impuretés dans un fluide (1), le fluide (2) pollué par des particules étant véhiculé par une première pompe (3) doseuse à un dispositif (4) de mesure de la pollution ou de la densité des particules dans le fluide pollué, **caractérisé en ce que** l'on mélange le fluide (2) pollué par les particules, avant l'entrée dans le dispositif (4) de mesure des impuretés, à du fluide (5) épuré en un rapport de mélange défini, on mesure la densité des particules ou la pollution du fluide mélangé, et on détermine, par une unité (7) de calcul, la densité de particules ou la pollution du fluide (2) pollué.

2. Procédé de détection de particules pour détecter des impuretés dans un fluide (1) suivant la revendication 1, **caractérisé en ce que** l'on mélange entre eux le fluide (2) pollué et le fluide (5) épuré dans un dispositif (8) de mélange.

3. Procédé de détection de particules pour détecter des impuretés dans un fluide (1) suivant la revendication 2, **caractérisé en ce que** le dispositif (8) de mélange est un récipient (9) et/ou une vanne de mélange.

4. Installation de détection de particules pour effectuer le procédé de détection de particules suivant l'une des revendications 1 à 3, dans laquelle le fluide (2) pollué par des particules peut être véhiculé par une première pompe (3) doseuse à un dispositif (4) de mesure de la pollution ou de la densité des particules dans le fluide (2) pollué, **caractérisée en ce que** le fluide (2) pollué par les particules peut, avant l'entrée dans le dispositif (4) de mesure de la pollution ou de la densité de particules, être mélangé à du fluide (5) épuré en un rapport de mélange défini, **en ce que** la densité de particules ou la pollution du fluide (6) mélangé peut être mesurée par le dispositif (4) de mesure de la pollution ou de la densité de particules, et la densité de particules ou la pollution du fluide (2) pollué peut être déterminée par une unité (7) de calcul, et **en ce qu'**il est prévu un circuit (11) de dosage, par lequel du fluide (2) pollué peut être véhiculé par la première pompe (3) doseuse au dispositif (4) de mesure des impuretés, dans laquelle il est prévu une première vanne (V₁) en aval de la première pompe (3) doseuse, et dans laquelle, par le circuit (11) de dosage, du fluide (2) pollué peut, en fonction de la position de commutation de la vanne (V₁), être véhiculé soit dans un récipient (9), soit en direction d'une liaison (18) à un système hydraulique principal.

5. Installation de détection de particules suivant la revendication 4, **caractérisée en ce qu'**un circuit (12) principal comprend une deuxième pompe (13) doseuse, qui véhicule le fluide (2) pollué et le fluide (5) épuré au dispositif (4) de mesure de la pollution, dans laquelle il est prévu une troisième vanne (V₃) et une quatrième vanne (V₄) et, en fonction de la position de commutation des vannes (V₃, V₄) du circuit (12) principal, ou bien un mode (R) d'épuration ou bien un mode (M) de mélange pour du fluide (5) épuré et du fluide (2) pollué ou bien un mode (MM) de mesure ou bien un mode de vidange du circuit (11) de dosage et/ou du circuit (12) principal peut être effectué.

6. Installation de détection de particules suivant la revendication 5, **caractérisée en ce qu'**il est prévu une combinaison du circuit (11) de dosage et du circuit (12) principal, de manière à pouvoir faire fonctionner le circuit (11) de dosage dans un mode d'attente, et le circuit principal dans un mode (R) d'épuration.

7. Installation de détection de particules suivant la revendication 6, **caractérisée en ce que** la première pompe (3) de dosage est en fonctionnement, la première vanne (V₁) se trouve en une position de commutation, dans laquelle une liaison fluidique avec le système hydraulique principal est établie, et les deuxième, troisième et quatrième vannes (V₂, V₃, V₄) ont une position de commutation telle, qu'un courant de fluide, ayant du fluide (5) épuré, arrive du récipient (9), en passant par la deuxième pompe (13) doseuse, et en étant conduit par la troisième et à la quatrième vanne (V₃, V₄), à un filtre (19) et est retourné au récipient (9).

8. Installation de détection de particules suivant la revendication 5, **caractérisée en ce qu'**il est prévu une combinaison du circuit (11) de dosage et du circuit (12) principal, de manière à ce que le circuit (11) de dosage soit dans un mode (5) de préparation, pour l'envoi de fluide (2) pollué au dispositif (8) de mélange, et le circuit (12) principal dans un mode (M) de mélange de fluide (2) pollué et de fluide épuré.

9. Installation de détection de particules suivant la revendication 8, **caractérisée en ce que** la première pompe (3) de dosage est en fonctionnement, et la première vanne (V₁) est dans une position de commutation, dans laquelle du fluide (2) pollué est conduit au récipient (9), dans laquelle la deuxième vanne (V₂), ainsi que la troisième vanne (V₃) et la quatrième vanne (V₄) sont dans une position de commutation, de manière à ce qu'un courant de fluide passe de la deuxième pompe (3) doseuse au dispositif (8) de mélange, en contournant le dispositif (4) de mesure de la pollution du fluide, ainsi qu'en contournant le filtre (9).

10. Installation de détection de particules suivant la revendication 5, **caractérisée en ce qu'**il est prévu une combinaison du circuit (11) de dosage et du circuit (12) principal, de manière à ce que le circuit (11) de dosage se trouve dans un mode (Z) d'envoi du fluide (2) pollué et le circuit (12) principal dans un mode (M) de mélange du fluide (2) pollué et du fluide (5) épuré.

11. Installation de détection de particules suivant la revendication 10, **caractérisée en ce que** la première pompe (2) de dosage est en fonctionnement et la première vanne (V₁) véhicule le fluide (2) pollué dans le dispositif (8) de mélange, et la position de commutation de la deuxième vanne (V₂), de la troisième vanne (V₃) et de la quatrième vanne (V₄) est telle, que le fluide (6) mélangé est conduit, en contournant le dispositif (4) de mesure de la pollution, du dispositif (8) de mélange à nouveau à ce dispositif (8) de mélange.

12. Installation de détection de particules suivant la revendication 5, **caractérisée en ce qu'**il est prévu une combinaison du circuit (11) de dosage et du circuit (12) principal, de manière à ce que le circuit (11) de dosage se trouve dans un mode (V) de préparation, pour envoyer du fluide pollué au dispositif (8) de mélange et de manière à ce que le circuit (12) principal se trouve dans un mode (MM) de mesure.

13. Installation de détection de particules suivant la revendication 12, **caractérisée en ce que** la première pompe (3) doseuse est en fonctionnement et, par la première vanne (V₁), le fluide (2) pollué est conduit au système hydraulique principal, la deuxième vanne (V₂) étant dans une position d'arrêt, de sorte que du fluide (6) mélangé passe dans le dispositif (4) de mesure de la pollution et la troisième vanne (V₃), ainsi que la quatrième vanne (V₄) sont dans une position de commutation, de manière à retourner du fluide mesuré au récipient (9).

14. Installation de détection de particules suivant la revendication 5, **caractérisée en ce qu'**il est prévu une combinaison du circuit (11) de dosage et du circuit (12) principal, de manière à ce que le fluide (2) pollué ne soit pas mis en mouvement dans le circuit (11) de dosage et de manière à ce que le fluide épuré (5) et/ou mélangé (6) puisse être vidangé du dispositif (8) de mélange dans le circuit (12) principal.

15. Installation de détection de particules suivant la revendication 14, **caractérisée en ce que** la deuxième vanne (V₂) prend une position de commutation telle, que du fluide (2) pollué est conduit à la troisième vanne (V₃) et, de la troisième vanne (V₃), le fluide (2) est conduit par l'intermédiaire de la liaison (18), au système hydraulique principal, la quatrième vanne (V₄) prenant une position de commutation d'arrêt, qui rend possible de vidanger le circuit (11) de dosage et le circuit (12) principal, la vanne (V₁) faisant passer du fluide pollué pareillement dans la liaison (8) du système hydraulique principal.

16. Installation de détection de particules suivant l'une des revendications 4 à 15, **caractérisée en ce que** l'installation a un élément (CS) de capteur, comme par exemple un capteur optique ou un capteur Hall, qui détermine la quantité des particules de pollution et la transmet à un dispositif de commande et/ou de régulation, qui commande le circuit (11) de dosage et le circuit (12) principal, au moyen des vannes (V₁, V₂, V₃, V₄) ainsi que des pompes (3, 13) de dosage.

17. Installation de détection de particules suivant l'une des revendications 7 à 16, **caractérisée en ce que** l'installation a un point de collecte pour collecter des impuretés, et un aimant permanent de production d'un champ magnétique au point de collecte et une bobine, dans laquelle on alimentant en courant la bobine, un élément de l'installation (14) peut être déplacé et par le déplacement de l'élément, l'intensité du champ magnétique au point de collecte peut être modifiée.
